# EUROPEAN PATENT APPLICATION

(11) **EP 4 083 206 A1**
(43) Date of publication of application: **02.11.2022**
(21) Application number: 21851659.9
(22) Date of filing: 16.12.2021
(51) Int. Cl.: C12N 15/11, C12N 15/113, C12P 19/34, C12P 21/02

(54) **TRANSLATION PROMOTER, TRANSLATION TEMPLATE MRNA, TRANSCRIPTION TEMPLATE DNA, METHOD FOR PRODUCING TRANSLATION TEMPLATE MRNA, AND METHOD FOR PRODUCING PROTEIN**

(30) Priority: 03.03.2021 JP 2021033276
(71) Applicant: Nuprotein Co., Ltd., Tokushima-shi, Tokushima 770-0942 (JP)
(72) Inventor: MINAMI Satori, Nishinomiya-shi Hyogo 6620066 (JP); ITAYA Tomotaka, Tokushima-shi Tokushima 7700942 (JP); TADA Hiroaki, Tokushima-shi Tokushima 7700944 (JP); MINAMI Masataka, Nishinomiya-shi Hyogo 6620066 (JP)
(74) Representative: Heyerhoff Geiger & Partner Patentanwälte PartGmbB
(86) International application number: PCT/JP2021/046607
(87) International publication number: WO 2022/185664

(57) **Abstract**

Provided is a translation enhancer that can shorten the 5'UTR. The object is achieved by a translation enhancer used in a cell-free protein synthesis system, the translation enhancer is a combination of a first sequence corresponding to nucleic acids as a 5' untranslated region connected adjacent to a 5' terminal of a code region that codes the amino acid sequence of the target protein, and a second sequence corresponding to nucleic acids as a 3' untranslated region connected adjacent to a 3' terminal of a code region that codes the amino acid sequence of the target protein, and the first sequence and the second sequence have complementary sequences that form conjugation.

## Description

### [Technical Field]

The disclosure in the present application relates to a translation enhancer, translation template mRNA, transcription template DNA, a method of producing translation template mRNA, and a method of producing a protein.

### [Background Art]

A synthesis system to synthesize a protein in a cell-free manner is a protein synthesis system to prepare a medium containing intracellular element related to protein synthesis and perform a procedure from transcription to translation of template DNA in a cell-free manner. A variety of such cell-free protein synthesis systems are known. As such synthesis systems, there are a system to apply template DNA, which is a transcription template, to a medium to synthesize a protein that is a final product and a system to apply mRNA, which is a translation template, to a medium to synthesize a protein.

In both of these systems, it is required to synthesize transcription template DNA as an initial raw material. Typically, synthesis of transcription template DNA is to, first, clone cDNA that codes a protein to be synthesized, integrate the cloned cDNA into plasmid, and build a DNA region including a promoter, a code region, a terminator, or the like used for expression. Template DNA is then obtained by cutting out such a DNA region from the plasmid or directly performing a PCR on the plasmid.

It is considered that the structure of template DNA affects the expression efficiency in a cell-free protein synthesis system, and various attempts have been made to vectors that build an expression cassette. For example, it is reported that a particular translation enhancement sequence is introduced to a 5' untranslated region (5' UTR) (Patent Literatures 1 and 2). Further, it is also disclosed that an elongated 3' untranslated region (3' UTR) leads to a longer life time of mRNA, which is a translation template, and higher translation efficiency (Patent Literature 3). It is also reported that the 3' UTR of mRNA has preferably 1000 bases or more (Patent Literatures 4 and 5).

Further, use of the 3' UTR is stated also for a cell-free protein synthesis system using a yeast extract (Non-Patent Literature 1).

On the other hand, it is also known that transcription template DNA can be acquired via a nucleic acid amplification reaction by using a 3' UTR having 200 bases or less and mRNA and a protein can be efficiently acquired by applying the transcription template DNA to a cell-free protein synthesis system (see Patent Literature 6).

In cell-free protein synthesis, it is required to synthesize translation template mRNA from transcription template DNA. In terms of mRNA synthesis efficiency, it is preferable that the 3' UTR that is not related to synthesis of a target protein be shorter. However, it is preferable that the synthesis efficiency of a target protein be higher. Thus, it is known that, (1) as nucleic acids of a 3' UTR connected adjacent to the 3' terminal of the code region that codes the amino acid sequence of a target protein, (2) a first region made of a sequence of 10 to 40 nucleic acids adjacent to the 3' terminal of the code region and a second region made of a poly-A sequence having continuous 2 to 40 As connected to the first region are included, (3) the first region has hairpin structure, and thereby (4) the synthesis efficiency of the target protein is improved even with a relatively short 3' UTR.

### [Citation List]

### [Patent Literature]

Patent Literature 1: Japanese Patent Application Laid-Open No. 2009-72207
Patent Literature 2: Japanese Patent Application Laid-Open No. 2013-158342
Patent Literature 3: Japanese Patent Application Laid-Open No. 2007-97438
Patent Literature 4: Japanese Patent Application Laid-Open No. 2008-35701
Patent Literature 5: Japanese Patent Application Laid-Open No. 2005-247857
Patent Literature 6: International Publication No. 2016/143799
Patent Literature 7: Japanese Patent No. 6738111

### [Non-Patent Literature]

Non-Patent Literature 1: Rui Gan et al., "A combined cell-free transcription-translation system from Saccharomyces cerevisiae for rapid and robust protein synthe", Biotechonology Journal, 2014, 9, 641-651

### [Summary of Invention]

### [Technical Problem]

As disclosed in Patent Literatures 1 to 7 and Non-Patent Literature 1, various studies have been made for the 5' UTR and the 3' UTR in the field of cell-free protein synthesis. However, while introduction of a particular translation enhancement sequence to the 5' UTR is known as disclosed in Patent Literatures 1 and 2, there is no known method for shortening the 5' UTR.

The disclosure in the present application has been made in order to solve the above problem of the conventional art, and according to a thorough study, it has been newly found that, (1) by focusing on a combination of a first sequence that is a UTR on the 5' terminal side and a second sequence that is a UTR on the 3' terminal side of a code region that codes an amino acid sequence of a target protein and (2) by designing the combination so that the first sequence and the second sequence can form conjugation, it is possible to shorten the 5' UTR.

That is, an object of the disclosure of the present application is to provide a translation enhancer, translation template mRNA, transcription template DNA, a method of producing translation template mRNA, and a method of producing a protein that can synthesize a target protein even with a relatively short 5' UTR.

### [Solution to Problem]

The disclosure of the present application relates to a translation enhancer, translation template mRNA, transcription template DNA, a method of producing translation template mRNA, and a method of producing a protein illustrated below.
(1) A translation enhancer used in a cell-free protein synthesis system,
   wherein the translation enhancer is a combination of
   a first sequence that is a nucleic acid as a 5' untranslated region connected adjacent to a 5' terminal of the code region that codes the amino acid sequence of the target protein, and
   a second sequence that is a nucleic acid as a 3' untranslated region connected adjacent to a 3' terminal of the code region that codes the amino acid sequence of the target protein,
   wherein the first sequence and the second sequence have complementary sequences that form conjugation.
(2) The translation enhancer according to (1) above, wherein the length of nucleic acids of the first sequence is greater than or equal to 3.
(3) The translation enhancer according to (1) or (2) above, wherein the length of nucleic acids of the second sequence is greater than or equal to 20.
(4) The translation enhancer according to any one of (1) to (3) above, wherein the second sequence further includes a poly-A sequence in which adenines (A) are continuous on the 3' side from the conjugation.
(5) The translation enhancer according to any one of (1) to (4) above, wherein the first sequence and/or the second sequence includes one or more stems or stem loops.
(6) The translation enhancer according to any one of (1) to (5) above, wherein a sum of the number of nucleic acids on the 3' side from the conjugation of the first sequence and the number of nucleic acids on the 5' side from the conjugation of the second sequence is less than or equal to 25.
(7) Translation template mRNA including:
   the translation enhancer according to any one of (1) to (6) above; and
   a code region that codes an amino acid sequence of a target protein and is arranged between the first sequence and the second sequence.
(8) Transcription template DNA used in a cell-free protein synthesis system, the transcription template DNA including:
   a promoter region,
   a code region that codes the amino acid sequence of the target protein,
   the translation enhancer according to any one of (1) to (6) above,
   wherein the first sequence of the translation enhancer is arranged between the promoter region and the code region, and,
   the second sequence of the translation enhancer is connected adjacent to a 3' terminal of the code region.
(9) A method of producing translation template mRNA used in a cell-free protein synthesis system, the method comprising a step of:
   synthesizing translation template mRNA by using the transcription template DNA according to (8) above in the absence of cells and under the presence of an element used for transcribing transcription template DNA to mRNA.
(10) A method of producing a protein, the method comprising a step of:
   synthesizing a protein by using the translation template mRNA according to (7) above or translation template mRNA produced in the method of producing translation template mRNA according to (9) above in the absence of cells and under the presence of an element used for translating translation template mRNA into a protein.

The disclosure of the present application also relates to translation template mRNA, transcription template DNA, a method of producing translation template mRNA, and a method of producing a protein illustrated below.
(1) Translation template mRNA including:
   a translation enhancer; and
   a code region that codes an amino acid sequence of a target protein;
   wherein the translation enhancer is a combination of
   a first sequence that is a nucleic acid as a 5' untranslated region connected adjacent to a 5' terminal of the code region that codes the amino acid sequence of the target protein, and
   a second sequence that is a nucleic acid as a 3' untranslated region connected adjacent to a 3' terminal of the code region that codes the amino acid sequence of the target protein,
   wherein the first sequence and the second sequence form conjugation when the translation template mRNA is analyzed by MXfold, and
   wherein a sum of the number of nucleic acids on the 3' side from the conjugation of the first sequence and the number of nucleic acids on the 5' side from the conjugation of the second sequence is less than or equal to 25.
(2) The translation template mRNA according to (1) above, wherein the length of nucleic acids of the first sequence is greater than or equal to 3.
(3) The translation template mRNA according to (1) above, wherein the length of nucleic acids of the second sequence is greater than or equal to 20.
(4) The translation template mRNA according to (2) above, wherein the length of nucleic acids of the second sequence is greater than or equal to 20.
(5) The translation template mRNA according to any one of (1) to (4) above, wherein the second sequence further includes a poly-A sequence in which adenines (A) are continuous on the 3' side from the conjugation.
(6) The translation template mRNA according to any one of (1) to (4) above, wherein the first sequence and/or the second sequence includes one or more stems or stem loops.
(7) The translation template mRNA according to (5) above, wherein the first sequence and/or the second sequence includes one or more stems or stem loops.
(8) Transcription template DNA used in a cell-free protein synthesis system, the transcription template DNA including:
   a region that codes the translation template mRNA according to any one of (1) to (7) above; and
   a promoter region arranged at the 5' terminal of the first sequence.
(9) A method of producing translation template mRNA used in a cell-free protein synthesis system, the method comprising a step of:
   synthesizing translation template mRNA by using the transcription template DNA according to (8) above in the absence of cells and under the presence of an element used for transcribing transcription template DNA to mRNA.
(10) A method of producing a protein, the method comprising a step of:
   synthesizing a protein by using the translation template mRNA according to any one of (1) to (7) above or translation template mRNA produced in the method of producing translation template mRNA according to (9) above in the absence of cells and under the presence of an element used for translating translation template mRNA into a protein.

### [Advantageous Effect]

The use of the translation enhancer disclosed in the present application enables synthesis of a target protein even with a relatively short 5' UTR.

### [Brief Description of Drawings]

[FIG. 1] FIG. 1 is a schematic diagram illustrating an overview of a translation enhancer 1 and translation template mRNA 10.
[FIG. 2] FIG. 2 is a schematic diagram illustrating an overview of conjugation C of the translation enhancer 1 and the translation template mRNA 10.
[FIG. 3] FIG. 3 is a schematic diagram illustrating the number of nucleic acids of the translation enhancer 1 and the translation template mRNA 10.
[FIG. 4] FIG. 4 is a schematic diagram illustrating an optional additional configuration of the translation enhancer 1 and the translation template mRNA 10.
[FIG. 5] FIG. 5 is a schematic diagram illustrating an overview of transcription template DNA 100.
[FIG. 6] FIG. 6 is a graph illustrating results of fluorometry of GFP-HIS synthesized in Examples 1 to 19 and Comparative example 1 by relative values to Comparative example 1 when the measurement value of Comparative example 1, Ω, is defined as 100.
[FIG. 7A] FIG. 7A is a diagram in which translation template mRNA of Examples 1 and 3 was analyzed by using secondary structure prediction software MXfold and the secondary structure was visualized by using forna.
[FIG. 7B] FIG. 7B is a diagram in which translation template mRNA of Examples 18 and 19 was analyzed by using secondary structure prediction software MXfold and the secondary structure was visualized by using forna.
[FIG. 8] FIG. 8 is a diagram in which translation template mRNA of Examples 20, 21, and 29 was analyzed by using secondary structure prediction software MXfold and the secondary structure was visualized by using forna.
[FIG. 9] FIG. 9 is a graph illustrating results of fluorometry of GFP synthesized in Examples 20 to 29 by relative values to Example 29 when the measurement value of Example 29 to be compared is defined as 100.
[FIG. 10] FIG. 10 is a graph illustrating results of fluorometry of GFP synthesized in Examples 29 and 30 and Comparative example 2 by relative values to Comparative example 2 when the measurement value of Comparative example 2 is defined as 100.

### [Description of Embodiments]

A translation enhancer, translation template mRNA, transcription template DNA, a method of producing translation template mRNA, and a method of producing a protein disclosed in the present application will be described below in detail. Note that the following description is provided for easier understanding, and the technical scope disclosed in the present application is not limited to the following description. Obviously, the disclosed technique can be suitably changed to those other than the following illustrative examples within a scope not compromising the spirit disclosed in the present application.

### [Translation Enhancer]

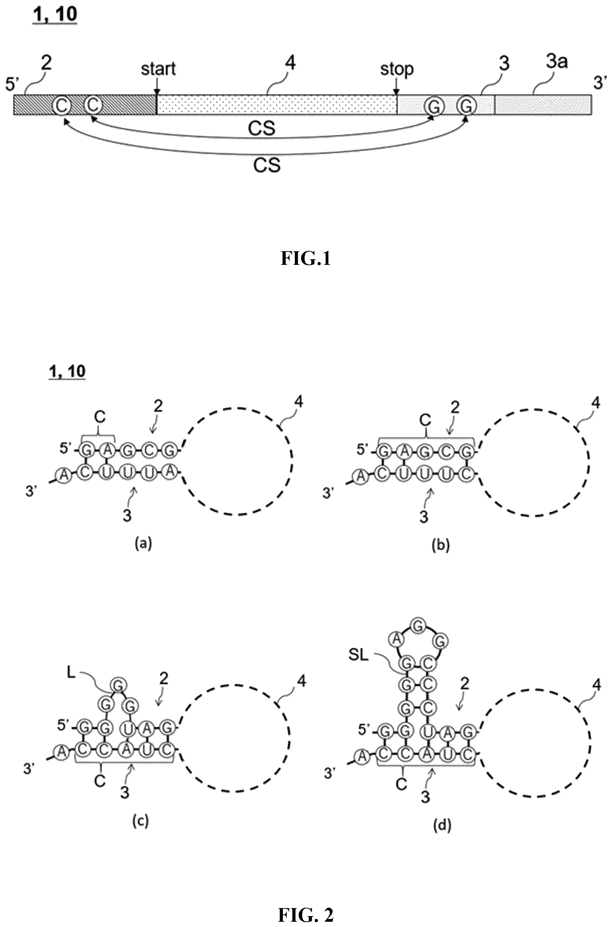

The embodiment of a translation enhancer will be described with reference to FIG. 1 and FIG. 2. FIG. 1 is a schematic diagram illustrating an overview of a translation enhancer. FIG. 2 is a schematic diagram illustrating an overview of conjugation of the translation enhancer. A translation enhancer 1 is a combination of a first sequence 2 corresponding to nucleic acids as a 5' untranslated region and a second sequence 3 corresponding to nucleic acids as a 3' untranslated region. The first sequence 2 is connected adjacent to the 5' terminal (start codon) of a code region 4 that codes the amino acid sequence of a target protein. The second sequence 3 is connected adjacent to the 3' terminal (stop codon) of a code region 4 that codes the amino acid sequence of the target protein. Note that, although a "stop codon" is not translated into an amino acid and may thus be considered as a 3' untranslated region, the "stop codon" is not included in the "second sequence 3" in the present specification. In other words, the "stop codon" is defined as a sequence of the code region 4. However, this does not exclude that a part of the stop codon forms a complementary pair. The first sequence 2 and the second sequence 3 have complementary sequences CS that form conjugation C.

The length of nucleic acids of the first sequence 2 is not particularly limited as long as conjugation with the second sequence 3 is formed. The length of nucleic acids of the first sequence 2 can be, for example, 1 or longer, 2 or longer, 3 or longer, 4 or longer, or 5 or longer. Note that, when mRNA that is a translation template is created by nucleic acid synthesis, the length of the first sequence 2 can be 1 or longer as described above. In contrast, when translation template mRNA is created by transcription from transcription template DNA, transcription of mRNA is started from a transcription starting point on the 3' side from a promoter. Therefore, when translation template mRNA is created from transcription template DNA, the first sequence 2 can be a length including at least the sequence located at the transcription starting point in accordance with the type of a promoter.

There is no upper limit in the length of the first sequence 2 as long as the first sequence 2 can form conjugation with the second sequence 3 and the length is within a range that enables synthesis of a target protein. Note that the translation enhancer 1 disclosed in the present application is characterized in that the first sequence 2 and the second sequence 3 are designed so as to have the complementary sequence CS forming conjugation and thereby the first sequence 2 can be shorter than the conventional 5' UTR. In terms of cost, a shorter UTR is preferable as long as a target protein can be synthesized. Therefore, the length of the nucleic acid of the first sequence 2 is not limited but may be, for example, 70 or less, 65 or less, 60 or less, 55 or less, 50 or less, 45 or less, 40 or less, 35 or less, 30 or less, 25 or less, 20 or less, 15 or less, or 10 or less.

The length of the nucleic acid of the second sequence 3 is not particularly limited as long as conjugation with the first sequence 2 can be formed. The length of the nucleic acid of the second sequence 3 can be, for example, 1 or greater, 2 or greater, 3 or greater, 4 or greater, or 5 or greater. Note that there are many reports to the effect that the length of the 3' UTR influences the synthesis efficiency of a target protein, as disclosed in Patent Literatures 3 to 7. Therefore, the nucleic acids of the second sequence 3 may be further elongated in terms of improvement of synthesis efficiency of a target protein and may be, for example, 20 or greater, 25 or greater, or 30 or greater. How much the length is to be may be suitably adjusted in accordance with the type of a target protein, the length of the first sequence 2, and the like.

There is no upper limit in the length of the second sequence 3 as long as the second sequence 3 can form conjugation with the first sequence 2 and the length is within a range that enables synthesis of a target protein. In terms of cost, however, a shorter UTR is preferable as long as a target protein can be synthesized, as with the first sequence 2. Therefore, the length of the nucleic acid of the second sequence 3 is not limited but may be, for example, 55 or shorter, 50 or shorter, 45 or shorter, 40 or shorter, 35 or shorter, 30 or shorter, or 25 or shorter.

Next, a specific example of conjugation will be described in more detail with reference to FIG. 2. First, in the present specification, a "conjugation" means a region where the first sequence 2 and the second sequence 3 form a complementary pair. Further, it can also be said that "conjugation" is a bonding portion for translation template mRNA to form a cyclic structure when the first sequence 2 is connected to the 5' terminal side of the code region 4 and the second sequence 3 is connected to the 3' terminal side of the code region 4 to create the translation template mRNA.

There is no particular limit in the number of complementary pairs (base pairs) forming the conjugation C. All the nucleic acids (bases) contained in the first sequence 2 and/or the second sequence 3 may form the conjugation, or some of the nucleic acids may form the conjugation. For example, in an example illustrated in FIG. 2(a), the conjugation C is formed by adjacent two sets of complementary pairs. Further, although two nucleic acids from the ends of the 5' terminal of the first sequence 2 form complementary pairs in the example illustrated in FIG. 2(a), nucleic acids forming a complementary pair may be located downstream of the 5' terminal. In other words, the first sequence 2 may contain a nucleic acid that does not form a complementary pair on the 5' side from (upstream of) the conjugation C. Also for the second sequence 3, in the same manner as the first sequence 2, the nucleic acid forming a complementary pair may form a complementary pair from the end of the 3' terminal or from the upstream of (on the 5' side from) the 3' terminal, as with the example illustrated in FIG. 2(a).

As illustrated in FIG. 2(b), the conjugation C may include a nucleic acid sequence that does not form a complementary pair. In such a case, "conjugation" means a region from one end to another of nucleic acids forming a complementary pair. In the example illustrated in FIG. 2(b), the first sequence 2 has five nucleic acids, and two nucleic acids from the 5' terminal and one nucleic acid from the 3' terminal form a complementary pair with the nucleic acids of the second sequence 3. Therefore, in the example illustrated in FIG. 2(b), all the nucleic acids of the first sequence 2 form the conjugation C, and the conjugation C includes a sequence that does not form a complementary pair. On the other hand, in the example illustrated in FIG. 2(b), some nucleic acids of the second sequence 3 form the conjugation C, and the conjugation C includes a sequence that does not form a complementary pair.

As illustrated in FIG. 2(c), the conjugation C may include one or more loops L. Although one loop L is formed in the first sequence 2 in the example illustrated in FIG. 2(c), the number of loops L may be two or greater. Further, although depiction is omitted, the loop L may be formed in only the second sequence 3, or the loop L may be formed in both the first sequence 2 and the second sequence 3.

As illustrated in FIG. 2(d), the conjugation C may include one or more stem loops SL. Although one stem loop SL is formed in the first sequence 2 in the example illustrated in FIG. 2(d), the number of stem loops SL may be two or greater. Further, although depiction is omitted, the stem loop SL may be formed in only the second sequence 3, or the stem loop SL may be formed in both the first sequence 2 and the second sequence 3.

The examples illustrated in FIG. 2(a) to FIG. 2(d) are mere illustrative examples of the arrangement of the conjugation C, and the arrangement is not limited to the illustrated arrangement. The examples illustrated in FIG. 2(a) to FIG. 2(d) may be combined such that the first sequence 2 has the loop L and the second sequence 3 has the stem loop SL, for example. Further, the examples illustrated in FIG. 2(a) to FIG. 2(d) represent the arrangement of the conjugation C. For example, when the first sequence 2 has a sequence other than the sequence forming the conjugation C, the sequence portion not involved in the formation of the conjugation C may include a loop or a stem loop. Similarly, when the second sequence 3 has a sequence other than the sequence forming the conjugation C, the sequence portion not involved in the formation of the conjugation C may include a loop or a stem loop. The sequences of the first sequence 2 and the second sequence 3 are not particularly limited as long as these sequences form the conjugation C and are within a range that enables the function as the translation enhancer 1.

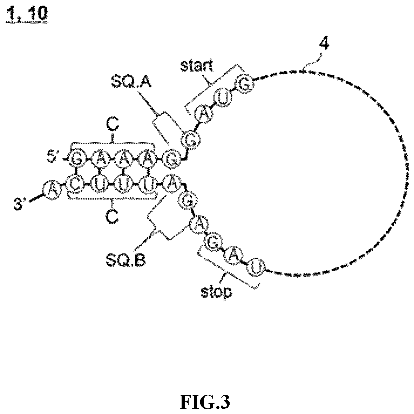

Further, as with the example illustrated in FIG. 2(a), when the first sequence 2 and the second sequence 3 include nucleic acids that do not form a complementary pair, the number of nucleic acids that do not form a complementary pair may be adjusted. The number of nucleic acids will be described with reference to FIG. 3. The nucleic acids of the first sequence 2 on the 3' side from the conjugation C, namely, the nucleic acid sequence up to a part immediately before the start codon are defined as a sequence A (SQ. A). The nucleic acids of the second sequence 3 on the 5' side from the conjugation C, namely, the nucleic acid sequence up to a part immediately before the stop codon are defined as a sequence B (SQ. B) . In the example illustrated in FIG. 3, the sequence A = 2, and the sequence B = 3. As illustrated in Examples described later, it is preferable that the number of the sequence A + the sequence B be smaller for expression of a protein. While not limited thereto, it is desirable that the number of the sequence A + the sequence B be 25 or less, 20 or less, 15 or less, 13 or less, 11 or less, 10 or less, or 6 or less, for example.

### [Optional Additional Configuration of Translation Enhancer 1]

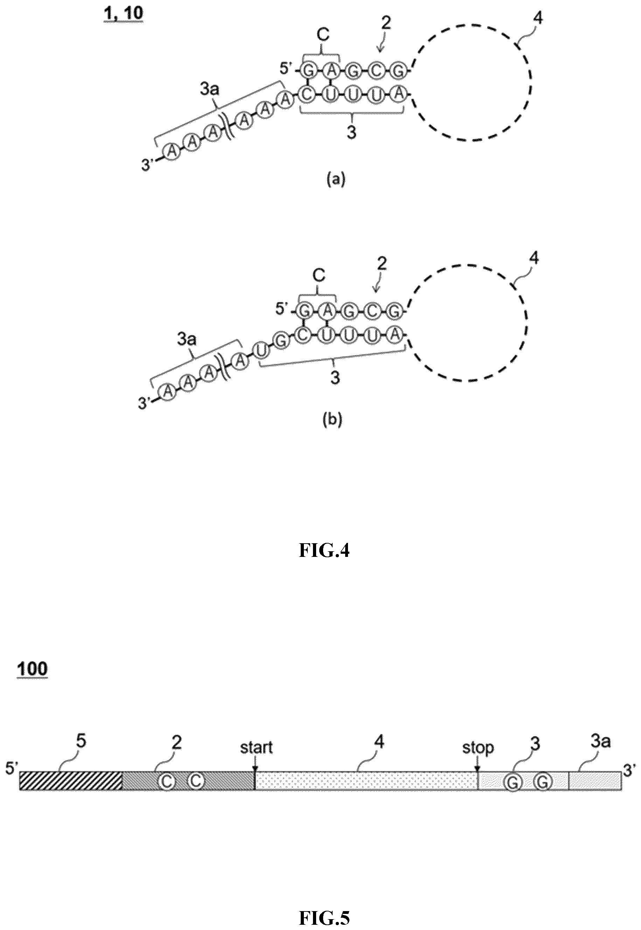

An optional additional configuration of the translation enhancer 1 will be described with reference to FIG. 1 and FIG. 4. FIG. 4 is a schematic diagram illustrating the optional additional configuration of the translation enhancer 1. The second sequence 3 may further include a poly-A sequence 3a in which adenines (As) are continuous on the 3' terminal side. The poly-A sequence 3a may be connected to a part immediately after the conjugation C of the second sequence 3 as illustrated in FIG. 4(a), or may be connected to the 3' terminal of the second sequence 3 subsequent to the conjugation C as illustrated in FIG. 4(b).

When the poly-A sequence 3a is connected on the 3' terminal side, improvement of translation efficiency of a target protein can be expected. The length of the poly-A sequence 3a may be suitably set within a range that improves the translation efficiency of a target protein. The length of the poly-A sequence 3a (the number of As) can be, but not limited to, 2 or greater, 3 or greater, 4 or greater, 5 or greater, 6 or greater, 7 or greater, 8 or greater, 9 or greater, 10 or greater, 11 or greater, 12 or greater, 13 or greater, 14 or greater, or 15 or greater, for example. On the other hand, while the upper limit is not particularly limited as long as it is within a range that enables synthesis of a target protein, the length of the poly-A sequence 3a (the number of As) can be 50 or less, 45 or less, 40 or less, 35 or less, or 30 or less in terms of primer design or in terms of cost.

### [Embodiment of Translation Template mRNA]

The embodiment of translation template mRNA will be described with reference to FIG. 1 to FIG. 4. In the translation template mRNA 10, the code region 4 that codes the amino acid sequence of a target protein is arranged between the first sequence 2 and the second sequence 3 of the translation enhancer 1 illustrated in FIG. 1 to FIG. 4. Inclusion of the translation enhancer 1 disclosed in the present application can shorten the 5' UTR of the translation template mRNA. Although depiction is omitted, the code region 4 may include one or more structures selected form a loop and a stem loop.

Further, when there is a nucleic acid sequence that does not form the conjugation C on the 3' side from the conjugation C of the first sequence 2, the code region 4 and the nucleic acid sequence that does not form the conjugation C may form one or more structures selected from a loop and a stem loop in a shared manner. Similarly, when there is a nucleic acid sequence that does not form the conjugation C on the 5' side from the conjugation C of the second sequence 3, the code region 4 and the nucleic acid sequence that does not form the conjugation C may form one or more structures selected from a loop and a stem loop in a shared manner.

When applied to translation template mRNA, the translation enhancer 1 is provided with a single-stranded nucleic acid on the 5' terminal side and the 3' terminal side of the code region 4. On the other hand, when applied to transcription template DNA described later, the translation enhancer 1 is provided with a double-stranded nucleic acid on the 5' terminal side and the 3' terminal side of the code region 4.

### [Transcription Template DNA]

The embodiment of transcription template DNA will be described with reference to FIG. 5. FIG. 5 is a schematic diagram illustrating an overview of transcription template DNA. Transcription template DNA 100 includes a promoter region 5, the code region 4 that codes the amino acid sequence of a target protein, and the translation enhancer 1 (the first sequence 2 and the second sequence 3 and, if necessary, the poly-A sequence 3a). Since the translation enhancer 1 has already been described, the promoter region 5 and the code region 4 will be described below in detail.

The sequence of the promoter region 5 is not particularly limited as long as the promoter region 5 functions as a transcription start part when a gene is transcribed, and a promoter sequence known in this technical field can be employed. A sequence forming the promoter region 5 may be a known T7 promoter sequence, a known SP6 promoter sequence, a known T3 promoter sequence, or the like, which are mere examples without limitation.

The code region 4 is not particularly limited as long as it is a nucleic acid sequence that codes the amino acid of a target protein and is connected so that the code region can be activated by the promoter region 5. Further, the first sequence 2 of the translation enhancer 1 is arranged between the promoter region 5 and the code region 4.

Note that, although the code region 4 is connected to a part immediately after the first sequence 2 in the example illustrated in FIG. 5, a sequence that codes a protein tag (N terminal protein tag) added to a target protein synthesized by the code region 4 may be connected to the first sequence 2 side of the code region 4. With inclusion of a protein tag sequence, a target protein can be synthesized as a fusion protein with the tag. Similarly, a sequence that codes a protein tag (C terminal protein tag) added to a target protein synthesized by the code region 4 may be connected to the second sequence 3 side of the code region 4. Any one of or both of the C terminal protein tag sequence and the N terminal protein tag sequence may be connected.

The C terminal protein tag and the N terminal protein tag may be, for example, a His tag, a GST tag, an MBP tag, an myc tag, a FLAG tag, or a BCCP tag. Further, a visually detectable tag may be, for example, Green Fluorescent Protein (GFP), Blue Fluorescent Protein (BFP), Cyan Fluorescent Protein (CFP), Red Fluorescent Protein (RFP), Yellow Fluorescent Protein (YFP), Enhanced Green Fluorescent Protein (EGFP), Enhanced Cyan Fluorescent Protein (ECFP), Enhanced Red Fluorescent Protein (ERFP), Enhanced Yellow Fluorescent Protein (EYFP), TetraMethyl-Rhodamine (TMR), luciferase, or the like. Note that the C terminal protein tags and the N terminal protein tags described above are mere examples, and other protein tags may be employed.

Note that the C terminal protein tag sequence and the N terminal protein tag sequence may be connected directly to the N terminal and/or the C terminal of any protein sequence or may be connected via a suitable linker sequence.

The transcription template DNA 100 is one of the elements used in a cell-free protein synthesis system described later. The transcription template DNA 100 may be of a linear form synthesized by a PCR or the like or otherwise may be a cyclic form such as plasmid. When the transcription template DNA 100 is in a DNA double stranded form, if the sense strand has the poly-A sequence 3a as the translation enhancer 1, the antisense strand will have a poly-T sequence, accordingly.

The transcription template DNA 100 can be acquired not only by a known chemical or genetical engineering method but also by utilizing a nucleic acid amplification reaction such as a PCR to use a gene or cDNA as a template. Further, the translation template mRNA 10 can be acquired by a known translation template mRNA synthesis method applied to a two-step method or the like.

### [Method of Producing Transcription Template DNA]

A method of producing the transcription template DNA 100 for a cell-free protein synthesis system can include a step of synthesizing the transcription template DNA 100 by performing a nucleic acid amplification reaction on DNA including the code region 4 of a target protein. The transcription template DNA 100 can be obtained by using suitably designed primer set to perform a nucleic acid amplification reaction of a PCR on the DNA including the code region 4 that codes the amino acid sequence of a target protein, for example.

Further, the transcription template DNA 100 can be acquired by using a vector. A template nucleic acid can be acquired by inserting, in a vector, the DNA including at least the code region 4 that codes the amino acid sequence of a protein. The vector created in such a way can be directly used as transcription template DNA, or a DNA fragment corresponding to the transcription template DNA may be cut out from the vector and the cut fragment may be used.

The transcription template DNA 100 may be applied to a cell-free protein synthesis system as a PCR reaction liquid (that is, without purification of the transcription template DNA 100), for example, or may be purified or the like if necessary and then applied to a cell-free protein synthesis system.

### [Method of Producing Translation Template mRNA]

A method of producing translation template mRNA for a cell-free protein synthesis system can include a step of synthesizing the translation template mRNA 10 by using the transcription template DNA 100 in the absence of cells and under the presence of an element used for transcribing transcription template DNA to mRNA. More specifically, for the transcription template DNA 100 derived from the PCR reaction liquid or the vector including the transcription template DNA 100, it is possible to obtain the translation template mRNA 10 by incubating RNA polymerase adapted to the promoter region 5 of the transcription template DNA 100, substrates for RNA synthesis (four types of ribonucleoside triphosphate), and the like, for example, at about 20 °C to 60 °C, preferably, about 30 °C to 42 °C for a suitable period of time under the composition containing components required for a transcription reaction. Note that the example described above represents a procedure to synthesize the translation template mRNA 10 from the transcription template DNA 100. Alternatively, the translation template mRNA 10 may be directly synthesized by nucleic acid synthesis depending on the length of mRNA.

The method of producing the translation template mRNA 10 can be implemented as a part of a transcription/translation system as a cell-free protein synthesis system or may be implemented as a step performed before application of the translation template mRNA 10 to the translation system. The reaction liquid of the translation template mRNA 10 obtained in such a way can be applied to the translation system.

### [Method of Producing Protein]

A method of producing a protein can include a step of synthesizing a protein by using the translation template mRNA 10 in the absence of cells and under the presence of an element used for translating translation template mRNA into a protein. A method of producing a protein can also include a step of synthesizing the translation template mRNA 10 by using the transcription template DNA 100 in the absence of cells and under the presence of an element used for transcribing transcription template DNA to mRNA. Furthermore, a method of producing a protein can also include a step of synthesizing the transcription template DNA 100 by performing a nucleic acid amplification reaction on DNA including the code region 4 of a target protein. The method of producing a protein disclosed in the present application uses the translation template mRNA 10 or the transcription template DNA 100 including the translation enhancer 1 and thus can shorten the 5' UTR.

While the embodiment disclosed in the present application will be specifically described below with Examples, these Examples are simply for the purpose of illustrating the embodiment. These Examples neither limit nor intend to restrict the scope of the invention disclosed in the present application.

### [EXAMPLES]

### <Examples 1 to 19, Comparative Example 1>

### (1) Structure of transcription template DNA

The overview of the transcription template DNA used in Examples 1 to 19 and Comparative example 1 will be described with reference to FIG. 5. As illustrated in FIG. 5, the transcription template DNA is formed of:
- 5' untranslated region (5' UTR): T7 promoter 5 + first sequence 2,
- code region 4: GFP-His, and
- 3' untranslated region (3' UTR): second sequence 3 (a length of nucleic acids of 20) + poly-A sequence 3a (a length of As of 15).

In Examples 1 to 19 and Comparative example 1, experiments were performed by changing the length and the type of the first sequence 2 of the translation enhancer (TRANSLATION ENHANCER). The sequences except for the first sequence 2 are as follows.

**[Table 1]**

| NAME | SEQUENCE | SEQ.ID. |
|---|---|---|
| T7 promoter | CCCGCGAAAT TAATACGACT CACTATA | 1 |
| GFP-His | ATGAGTAAAGGAGAAGAACTTTTCACTGGAGTTGTCCCAATTCTTGTTGAATTAGATGGTGATGTTAATGGGCACAA ATTTTCTGTCAGTGGAGAGGGTGAAGGTGATGCAACATACGGAAAACTTACCCTTAAATTTATTTGCACTACTGGAA AACTACCTGTTCCATGGCCAACACTTGTCACTACTTTCTCTTATGGTGTTCAATGCTTTTCAAGATACCCAGATCAT ATGAAACGGCATGACTTTTTCAAGAGTGCCATGCCCGAAGGTTATGTACAGGAAAGAACTATATTTTTCAAAGATGA CGGGAACTACAAGACACGTGCTGAAGTCAAGTTTGAAGGTGATACCCTTGTTAATAGAATCGAGTTAAAAGGTATTG ATTTTAAAGAAGATGGAAACATTCTTGGACACAAATTGGAATACAACTATAACTCACACAATGTATACATCATGGCA GACAAACAAAAGAATGGAATCAAAGTTAACTTCAAAATTAGACACAACATTGAAGATGGAAGCGTTCAACTAGCAGA CCATTATCAACAAAATACTCCAATTGGCGATGGCCCTGTCCTTTTACCAGACAACCATTACCTGTCCACACAATCTG CCCTTTCGAAAGATCCCAACGAAAAGAGAGACCACATGGTCCTTCTTGAGTTTGTAACAGCTGCTGGGATTACACAT GGCATGGATGAACTATACAAAGGTCTCCAGCAGGGAGGTACTCATCATCATCATCATCATTAG | 2 |
| 3^{.}UTR | AATAA GTGCTCGGGCGGGCC AAAAAAAAAA AAAAA | 3 |

Table 2 lists the first sequence (First SEQ) of Examples 1 to 19 and Comparative example 1. In Examples 1 to 19, the length of the first sequence 2 was changed from 5 to 33. Note that Comparative example 1, Ω, is a known 5' UTR translation enhancer (a length of nucleic acids of 66) derived from a tobacco mosaic virus, and the details thereof are disclosed in Japanese Patent Application Laid-Open No. 2013-503640.

**[Table 2]**

| NAME | SEQUENCE of first SEQ of TRANSLATION ENHANCER (5' to 3') | SEQ.ID. |
|---|---|---|
| 1-1-A(Example 1) | GGGCC | 4 |
| 1-2-A(Example 2) | GGGCCTGA | 5 |
| 1-3-A(Example 3) | GGGCCAAGA | 6 |
| 1-4-A(Exanple 4) | GGGCCAAAGA | 7 |
| 1-5-A(Example 5) | GGGCCAAAAGA | 8 |
| 1-6-A(Example 6) | GGGCCGAAAACA | 9 |
| 1-7-A(Example 7) | GGGCCGGAAAAGA | 10 |
| 1-8-A(Example 8) | GGGCCAGGAAAAGA | 11 |
| 1-9-A(Example 9) | GGGCCGAGGAAAAGA | 12 |
| 1-10-A(Example 10) | GGGCCAGAGGAAAAGA | 13 |
| 1-11-A(Example 11) | GGGCCGAAGAAGAGGAAAAGA | 14 |
| 1-12-A(Example 12) | GGGCCTTTTCAAGAAGAGGAAAAGA | 15 |
| 1-13-A(Example 13) | GGGCCCTTTTCAAGAAGAGGAAAAGA | 16 |
| 1-14-A(Example 14) | GGGCCACTTTTCAAGAAGAGGAAAAGA | 17 |
| 1-15-A(Example 15) | GGGCCCACI1WCAAGAAGAGGAAAAGA | 18 |
| 1-16-A(Example 16) | GGGCCTCACTTTTCAAGAAGAGGAAAAGA | 19 |
| 1-18-A(Example 17) | GGGCCGGTCACTTTTCAAGAAGAGGAAAAGA | 20 |
| 1-19-A(Example 18) | GGGCCAGGTCACTTTTCAAGAAGAGGAAAAGA | 21 |
| 1-20-A(Example 19) | GGGCCGAGGTCACTTTTCAAGAAGAGGAAAAGA | 22 |
| Ω (Comparative example 1) | GGGTATTTTTACAACAATTACCAACAACAACAAACAACAAACAACATTACAATTACTATTTACAATTACA | 23 |

### (2) Creation of transcription template DNA by PCR

For transcription template DNA, a set of primers listed in Table 3 (the forward side: FW-1-1-A, the reverse side: RV) were designed in Example 1, and the transcription template DNA was created by a PCR. Note that the primer was created by a custom synthesis service (Eurofins Genomics K.K.). The forward primers of Examples 2 to 19 used herein were forward primers in which the bold underline part (GGGCC) of FW-1-1-A in Table 3 below is replaced with sequences of Examples 2 to 19 in Table 2, respectively. The reverse primers of Examples 2 to 19 used herein were the same RV as that of Example 1. Comparative example 1, Ω, was created in the same manner as in Example 1 except that FW-Ω (SEQ. ID. 25) was used in the forward primer.

**[Table 3]**

| NAME | SEQUENCE | SEQ.ID. |
|---|---|---|
| FW-1-1-A | CCCGCGAAATTAATACGACTCACTATA**GGGCC**ATGAGTAAAGGAGAAGAACTTTTCACTGGAGTTGT | 24 |
| FW-Ω | CCCGCGAAATTAATACGACTCACTATAGGGTATTTTACAACAATTACCAACAACAACAAACAACAAACAACATTACA ATTACTATTTACAATTACAATGAGTAAAGGAGAAGAACTTTTCACTGGAGTTGT | 25 |
| RV | TTTTT TTTTTTTTTT GGCCCGCCCGAGCAC TTATT | 26 |

Table 4 lists compositions of a reaction solution of the PCR. Further, Table 5 lists reaction cycles. Note that a reagent and a machine used herein are as follows.
- PCR enzyme: KOD-Plus-Neo by TOYOBO CO., LTD.
- Thermal Cycler: Mastecycler X50s by Eppendorf

**[Table 4]**

| Reagents | Volume |
|---|---|
| 10× PCR buffer | 5 µL |
| 2 mM dNTPs | 5 µL |
| 25 mM MgSO₄ | 3 µL |
| 10 µM Fw primer | 1 µL |
| 10 µM Rv primer | 1 µL |
| plasmid | 1 ng |
| KOD Polymerase DNA | 1 µL |
| Ultra pure water | 50 µL |

**[Table 5]**

| | |
|---|---|
| 1.Predenature | 94°C, 2min |
| 2.Denature | 98°C 10 sec |
| 3. Annealing | 55°C 30 sec |
| 4. Extension | 68°C 1m30 sec (2-4 for 30 cycles) |

### (3) Transcription reaction

Next, the created transcription template DNA was used to create translation template mRNA. The transcription reaction was performed at 37 °C for 3 hours by using a reaction liquid indicated in Table 6 below of PSS4050 by NUProtein and using the previously created PCR reaction solution of 2.5 µl (containing transcription template DNA).

**[Table 6]**

| Reagents | Volume |
|---|---|
| 10× Transcription buffer | 2.5 µL |
| 25 mM NTP Mix | 2.5 µL |
| 100 mM DTT | 1.25 µL |
| T7 RNA Polymerase | 1 µL |
| PCR Product | 2.5 µL |
| RNAase free water | 15.25 µL |

Then, 4M ammonium acetate of 10 µL was added to the transcription reaction liquid of 25 µl, the mixture was well mixed, 100% ethanol of 100 µl was further added thereto, the mixture was inverted and centrifuged for several seconds by a desktop centrifuge, and the mixture was then allowed to stand still at -20 °C for 10 minutes. The mixture was then centrifuged (12,000 rpm, 15 minutes, 4 °C). After the supernatant was removed, the remaining solution was centrifuged for several seconds by using the desktop centrifuge. The supernatant was removed again, and the remaining solution was allowed to stand still until the precipitate was dried. Then, RNase free water (DEPC water) of 40 µL was added to the transcription reaction liquid of 25 µl, and the precipitate was well suspended by a chip. In accordance with the PSS4050 protocol, nucleic acid concentration measurement was performed so that the amount of mRNA in the translation solution of 110 µl was 35 µg, and the solution was filled up to 80 µl to have a translation template mRNA solution.

### (4) Translation reaction

Next, a translation reaction liquid having the following composition was used and put into an incubator at 16 °C to be reacted for 10 hours. Note that a composition liquid excluding the translation template mRNA of the following composition was prepared, this composition liquid was then warmed back to room temperature, the translation template mRNA was then added thereto, and the mixture was pumped so as not to be foamed and was reacted. PSS4050 by NUProtein was used for Wheat germ extract and Amino acid mix.

**[Table 7]**

| Reagents | Volume |
|---|---|
| Wheat germ extract | 20 µL |
| Amino acid mix | 40 µL |
| mRNA solution | 160 µL |

After the reaction, a reaction liquid was collected into an Eppendorf tube, and centrifuged (15,000 rpm, 15 minutes, 4 °C), and the supernatant was a GFP solution resulted after completion of translation.

### [Fluorometry of Synthesized GFP]

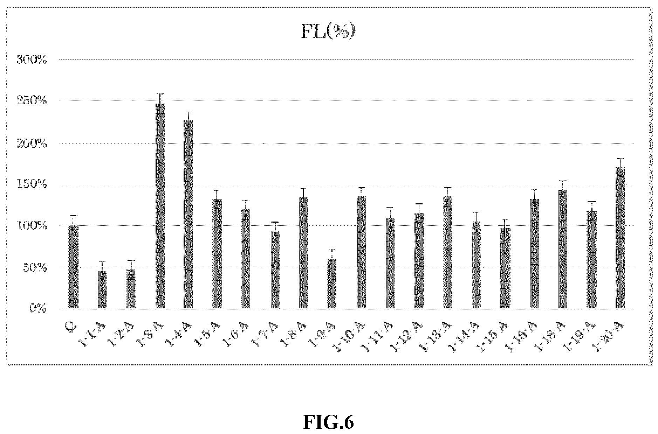

Fluorometry of the GFP synthesized in Examples 1 to 19 and Comparative example 1 was performed. A solution of 220 µl containing the synthesized GFP was used as a sample and irradiated with excitation light having a wavelength of 475 nm, and fluorescence from the GFP was measured by a plate reader (absorption filter with 500 to 550 nm) . A GloMax (registered trademark) plate reader (by Promega Corporation) was used as the plate reader. FIG. 6 illustrates measurement results. FIG. 6 is a graph illustrating the measurement values of Examples 1 to 19 by relative values to Comparative example 1 when the measurement value of Comparative example 1, Ω, is defined as 100.

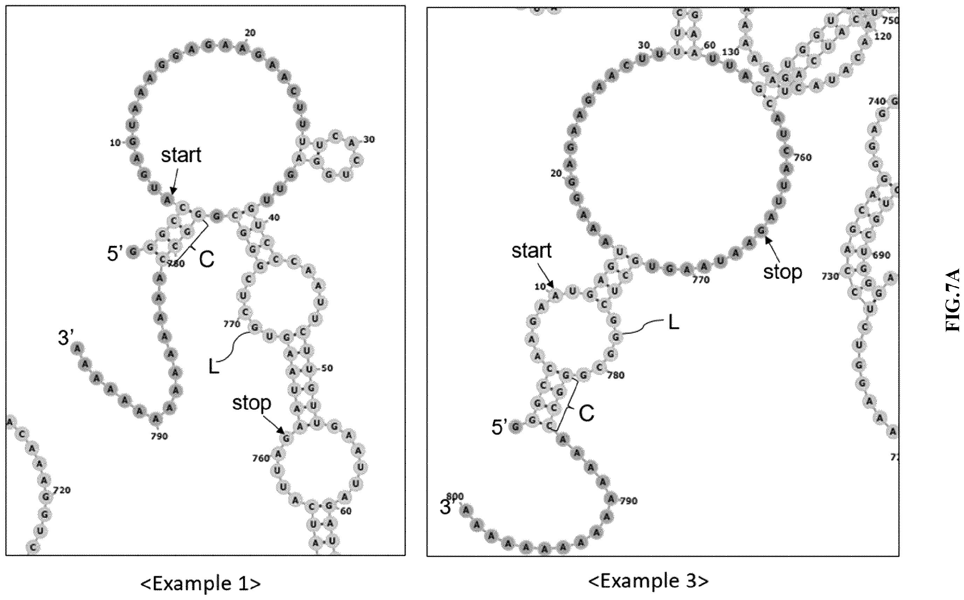

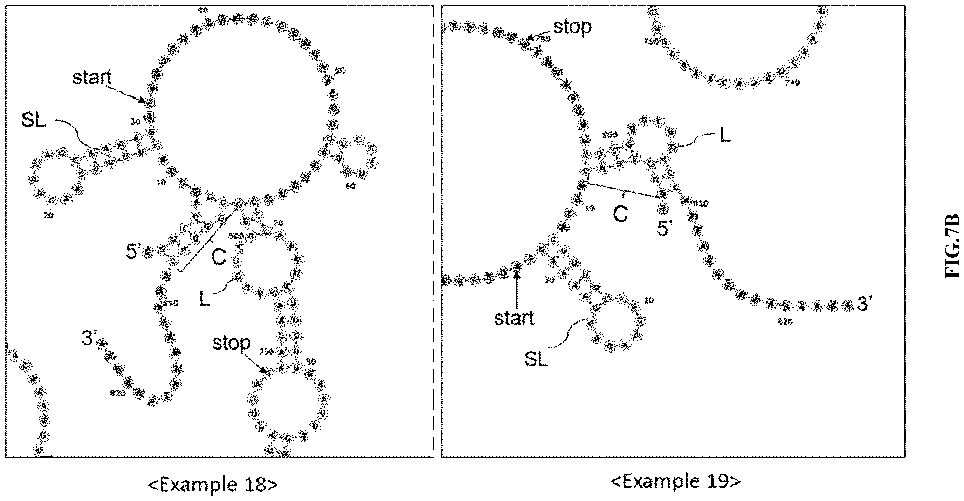

Further, among the translation template mRNA created in Examples, the translation template mRNA of Examples 1, 3, 18, and 19 was analyzed by using secondary structure prediction software MXfold (http://www.dna.bio.keio.ac.jp/mxfold/), and the secondary structure was visualized by using forna (http://rna.tbi.univie.ac.at/forna/). FIG. 7A and FIG. 7B are enlarged views of a part near the coupling part C of the visualized secondary structure of Examples 1, 3, 18, and 19.

Note that the arrow of "start" in FIG. 7A and FIG. 7B indicates "A" of "AUG" that is a start codon. The part on the 5' side from the "A" pointed by the arrow of "start" corresponds to the first sequence 2. The "stop" in FIG. 7A and FIG. 7B indicates "G" of "UAG" that is a stop codon. The part on the 3' side from the "G" pointed by the arrow of "stop" corresponds to the second sequence 3.

As is apparent from FIG. 7A and FIG. 7B, it was confirmed that, by designing the sequence of a translation enhancer so as to include a complementary sequence, it is possible for the first sequence 2 and the second sequence 3 to form the conjugation C. Further, it was confirmed that, by suitably adjusting the length and the sequence of the first sequence 2 and the second sequence 3, for example,
(1) it is possible to form the stem loop SL on the 3' side (code region 4 side) from the conjugation C by using the first sequence 2 alone (Examples 18 and 19),
(2) it is possible to form the loop L in the conjugation C (Example 19), and
(3) it is possible to form the loop L in mRNA with the first sequence 2 and/or the second sequence 3 cooperating with the code region 4 (Examples 1, 3, and 18) . Note that, for other Examples, although depiction of the secondary structure is omitted, it is apparent that the conjugation C can be formed, because all the Examples have the same nucleic acid sequence of five nucleic acids on the 5' terminal side of the first sequence 2 and all the Examples have the same second sequence 3, as indicated in Table 2.

As illustrated in FIG. 6, a protein can be synthesized from mRNA even when the length of the nucleic acids of the first sequence 2 is only five (1-1-A), and Example 3 having a length of nine (1-3-A) exhibited a translation enhancement effect that is about 2.5 times that of Ω known as the translation enhancer of the 5' UTR. It was confirmed from the above results that it is possible to shorten the 5' UTR by forming the conjugation C by using the 5' UTR and the 3' UTR. Note that, although the mechanism by which the 5' UTR can be shortened is not clear, it is inferred that, in view of the secondary structure of FIG. 7A and FIG. 7B, the positions of the start codon and the stop codon come closer to each other due to the formation of the conjugation C, and next translation may be started promptly after the end of previous translation.

### <Examples 20 to 29>

To confirm the above inference, an experiment to change the sum of the number of nucleic acids of the first sequence 2 from the conjugation C to the part immediately before the start codon and the second sequence 3 from the part immediately after the stop codon to the conjugation C was performed. Specifically, the first sequence 2 described in Examples 1 to 19 was formed of 10-nucleic acid sequence in Table 8 (SEQ. ID. 27). Further, the second sequence 3 was designed such that the portion of "GGGCC" forms conjugation and the portion of "AAAGA" does not form the conjugation C. That is, the number of nucleic acids from the conjugation C to the part immediately before the start codon of the first sequence 2 (sequence A) was fixed to five.

The sequence forming the conjugation C of the second sequence 3 was "GCCC" in Example 20 as illustrated in Table 8 and was designed such that the last G of the stop codon was included to form the conjugation C with "GGGCC" of the first sequence. Since the last G of the stop codon also forms the conjugation C, the number of nucleic acids from the part immediately after the stop codon to the conjugation C of Example 20 (sequence B) is 0.

In Example 21, the sequence forming the conjugation C of the second sequence 3 was "GGCCC", the conjugation C was formed together with "GGGCC" of the first sequence 2, and design was made such that the number of nucleic acids from the conjugation C to a part immediately before the stop codon (sequence B) had one of the adenines (A) (the bold underlined character A of SEQ. ID. 30 in Table 8). In Examples 22 to 28, since the length was adjusted by only addition of adenine (A) to the sequence B of Example 21, presentation of sequence table is omitted. The promoter used herein was the same as those in Examples 1 to 19, and the code region 4 used herein was GFP listed in Table 8.

**[Table 8]**

| NAME | SEQUENCE | SEQ.ID. |
|---|---|---|
| first. SEQ of TE | **GGGCC**AAAGA | 27 |
| GFP | ATGAGTAAAGGAGAAGAACTTTTCACTGGAGTTGTCCCAATTCTTGTTGAATTAGATGGTGATGTTAATGGGCACAA ATTTTCTGTCAGTGGAGAGGGTGAAGGTGATGCAACATACGGAAAACTTACCCTTAAATTTATTTGCACTACTGGAA AACTACCTGTTCCATGGCCAACACTTGTCACTACTTTCTCTTATGGTGTTCAATGCTTTTCAAGATACCCAGATCAT ATGAAACGGCATGACTTTTTCAAGAGTGCCATGCCCGAAGGTTATGTACAGGAAAGAACTATATTTTTCAAAGATGA CGGGAACTACAAGACACGTGCTGAAGTCAAGTTTGAAGGTGATACCCTTGTTAATAGAATCGAGTTAAAAGGTATTG ATTTTAAAGAAGATGGAAACATTCTTGGACACAAATTGGAATACAACTATAACTCACACAATGTATACATCATGGCA GACAAACAAAAGAATGGAATCAAAGTTAACTTCAAAATTAGACACAACATTGAAGATGGAAGCGTTCAACTAGCAGA CCATTATCAACAAAATACTCCAATTGGCGATGGCCCTGTCCTTTTACCAGACAACCATTACCTGTCCACACAATCTG CCCTTTCGAAAGATCCCAACGAAAAGAGAGACCACATGGTCCTTCTTGAGTTTGTAACAGCTGCTGGGATTACACAT GGCATGGATGAACTATACAAATAG | 28 |
| 3'UTR (Example 20) | **GCCC**AAAAAAAAAAAAAAA | 29 |
| 3 'UTR (Example 21) | **AGGCCC**AAAAAAAAAAAAAAA | 30 |

The overview of Examples 20 to 28 is described below. Each length of the sequence A is 5. The sequence B corresponds to the number of adenines (A).
- Example 20 (1-4-A/RV0): sequence B = 0, sequences A + B = 5
- Example 21 (1-4-A/RV1A): sequence B = 1, sequences A + B = 6
- Example 22 (1-4-A/RV5A): sequence B = 5, sequences A + B = 10
- Example 23 (1-4-A/RV10A): sequence B = 10, sequences A + B = 15
- Example 24 (1-4-A/RV15A): sequence B = 15, sequences A + B = 20
- Example 25 (1-4-A/RV20A): sequence B = 20, sequences A + B = 25
- Example 26 (1-4-A/RV30A): sequence B = 30, sequences A + B = 35
- Example 27 (1-4-A/RV40A): sequence B = 40, sequences A + B = 45
- Example 28 (1-4-A/RV50A): sequence B = 50, sequences A + B = 55

- As Example to be compared (Example 29, GFP-1 + PlyA), translation template mRNA was created in which (1) the number of nucleic acids of the first sequence 2 was 23, the number of nucleic acids forming the conjugation C of the first sequence 2 was 4, and the number of nucleic acids of the sequence A was 19 and (2) the number of nucleic acids of the second sequence 3 was 40 (including the poly-A sequence), the number of nucleic acids forming the conjugation C of the second sequence 3 was 4, and the number of nucleic acids of the sequence B was 21.

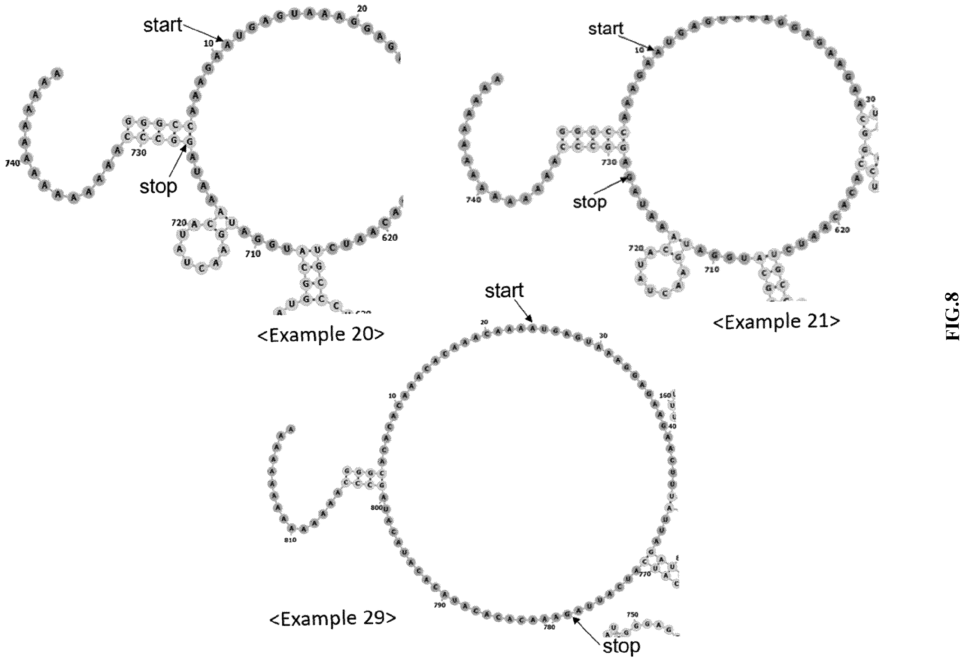

FIG. 8 is a diagram in which translation template mRNA created in Examples 20, 21, and 29 was analyzed by using secondary structure prediction software MXfold (http://www.dna.bio.keio.ac.jp/mxfold/) and the secondary structure is visualized by using forna (http://rna.tbi.univie.ac.at/forna/). In Examples 20, 21, and 29, it was confirmed that the numbers of nucleic acids in the sequence A and the sequence B were as designed. Although depiction is omitted, it was confirmed that each translation template mRNA created in Examples 22 to 28 was also as designed.

All the forward primers (FW-1-4-A) of Examples 20 to 28 used herein were the same. The reverse primer (RV-0) of Example 20 and the reverse primer (RV-1) of Example 21 are listed in Table 9. Note that the reverse primer of Examples 22 to 28 can be created by changing the length of "T" of "GGGCCTC" of RV-1 (the underlined part of SEQ. ID. 33 in Table 9). Therefore, listing of the reverse primers of Examples 22 to 28 is omitted. The forward primer (FW-GFP-1 + PlyA) and the reverse primer (RV-GFP-1 + PlyA) of Example 29 are listed in Table 9.

**[Table 9]**

| NAME | SEQUENCE | SEQ.ID. |
|---|---|---|
| FW-1-4-A | CCCGCGAAATTAATACGACTCACTATAGGGCCAAAGAATGAGTAAAGGAGAAGAACTTTTCACTGGAGTTGT | 31 |
| RV-0 | TTTTTTTTTTTTTTTGGGCCTATTTGTATAGTTCATCCATGCCATGTGTAATCCCAG | 32 |
| RV- 1 | TTTTTTTTTTTTTTTGGGCC**T**CTATTTGTATAGTTCATCCATGCCATGTGTAATCCCAG | 33 |
| FW-GFP-1+PlyA | CCCGTTAATACGACTCACTATAGGGCACACACAAACACAAACAAAATGAGTAAAGGAGAAGAACTTTTCACTGGAGT TGTCCC | 34 |
| RV-GFP-1+PlyA | TTTTTTTTTTTTTTTCCCGTTTGTTTGTGTTTGTGTGTTTCTATTTGTATAGTTCATCCATGCCATGTGTAATCCCA G | 35 |

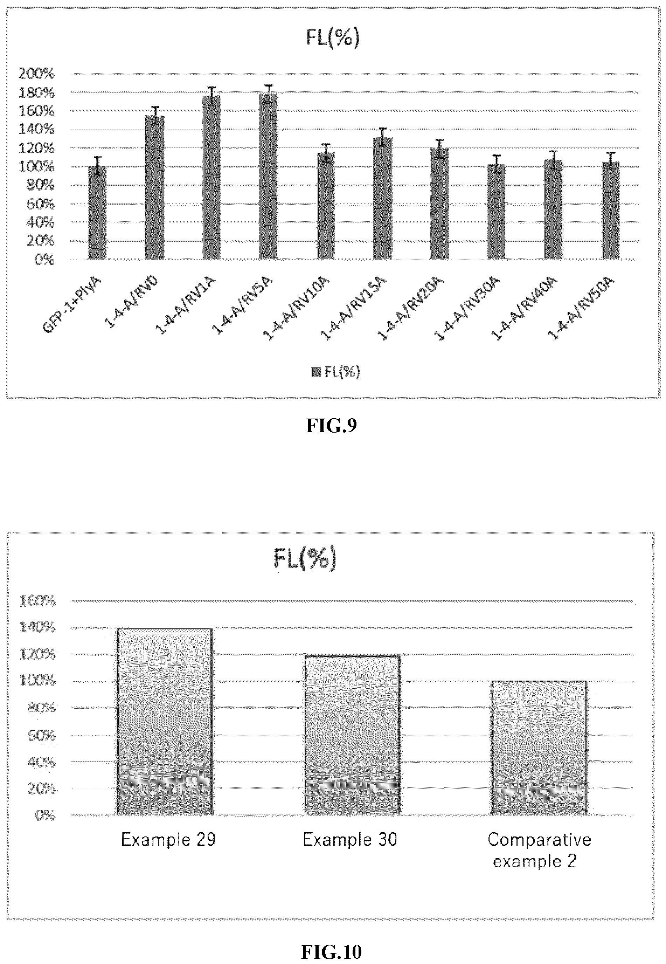

FIG. 9 illustrates fluorometry results of the synthesized GFP. FIG. 9 is a graph illustrating measurement values of Examples 20 to 28 by relative values to Example 29 when the measurement value to be compared (Example 29, GFP-1 + PlyA) is defined as 100. As is apparent from FIG. 9, it was confirmed that, when the number of sequences A and sequences B is small, in other words, when the start codon and the stop codon are arranged close to each other, the translation efficiency becomes higher.

### <Example 30, Comparative Example 2>

Next, an experiment with removal of the poly-A sequence on the 3' terminal side of the second sequence 3 of Example 29 was performed. Example 30 is the same as Example 29 except that the poly-A sequence of Example 29 was removed. In Comparative example 2, the translation enhancer disclosed in Japanese Patent Application Laid-Open No. 2003-556626 was used as the 5' UTR, and the poly-A sequence disclosed in International Publication No. 2016/143799 was used as the 3' UTR. Note that the 3' UTR of Comparative example 2 has the poly-A sequence connected to the part immediately after the stop codon and thus forms no conjugation. Table 10 lists the primers. Example 30 has the same FW as Example 29. The GFP fluorescent was measured by the same procedure as Examples described above except for the difference in the primer.

**[Table 10]**

| NAME | SEQUENCE | SEQ.ID. |
|---|---|---|
| RV-GFP-1-PlyA | GGGCTATGTATGTGTATGTGTGTTTCTATTTGTATAGTTCATCCATGCCATGTGTAATCCCAG | 36 |
| Comparative example 2 FW | CCCGTTAATACGACTCACTATAGGGCCCAACACCTAATAACATTCAATCACTCTTTCCACTAACCACCTATCTACAT CACCAATGAGTAAAGGAGAAGAACTTTTCACTGGAGTTGTCCC | 37 |
| Comparative example 2 RV | TTTTTTTTTTTTTTTCTATTTGTATAGTTCATCCATGCCATGTGTAATCCCAG | 38 |

FIG. 10 illustrates results of fluorometry on the synthesized GFP. Note that the measurement result of Example 29 is indicated together for comparison. FIG. 10 is a graph illustrating the result by relative values to Comparative example 2 when the measurement value of Comparative example 2 is defined as 100. As is apparent from FIG. 10, because the 5' UTR and the 3' UTR form conjugation, a more amount of the protein was synthesized than in the case of translation template mRNA combining known 5' translation enhancer and 3' translation enhancer. Further, it was confirmed that, in the translation enhancer disclosed in the present application, addition of a poly-A sequence to the 3' side of the second sequence 3 further improves the translation efficiency.

### [Industrial Applicability]

With the translation enhancer disclosed in the present application, the 5' UTR can be shortened. Therefore, the translation enhancer and thus the present disclosure are useful in industries such as a pharmaceutical industry, research institutes, or the like that require cell-free protein synthesis.

### [List of References]

- 1: translation enhancer
- 2: first sequence
- 3: second sequence
- 3a: poly-A sequence
- 4: code region
- 5: promoter region
- 10: translation template mRNA
- 100: transcription template DNA
- C: conjugation
- CS: complementary sequence
- L: loop
- SL: stem loop

## Claims

1. Translation template mRNA including:
a translation enhancer; and
a code region that codes an amino acid sequence of a target protein;
wherein the translation enhancer is a combination of
a first sequence that is a nucleic acid as a 5' untranslated region connected adjacent to a 5' terminal of the code region that codes the amino acid sequence of the target protein, and
a second sequence that is a nucleic acid as a 3' untranslated region connected adjacent to a 3' terminal of the code region that codes the amino acid sequence of the target protein,
wherein the first sequence and the second sequence form conjugation when the translation template mRNA is analyzed by MXfold, and
wherein a sum of the number of nucleic acids on the 3' side from the conjugation of the first sequence and the number of nucleic acids on the 5' side from the conjugation of the second sequence is less than or equal to 25.

2. The translation template mRNA according to claim 1, wherein the length of nucleic acids of the first sequence is greater than or equal to 3.

3. The translation template mRNA according to claim 1, wherein the length of nucleic acids of the second sequence is greater than or equal to 20.

4. The translation template mRNA according to claim 2, wherein the length of nucleic acids of the second sequence is greater than or equal to 20.

5. The translation template mRNA according to any one of claims 1 to 4, wherein the second sequence further includes a poly-A sequence in which adenines (A) are continuous on the 3' side from the conjugation.

6. The translation template mRNA according to any one of claims 1 to 4, wherein the first sequence and/or the second sequence includes one or more stems or stem loops.

7. The translation template mRNA according to claim 5, wherein the first sequence and/or the second sequence includes one or more stems or stem loops.

8. Transcription template DNA used in a cell-free protein synthesis system, the transcription template DNA including:
a region that codes the translation template mRNA according to any one of claims 1 to 7; and
a promoter region arranged at the 5' terminal of the first sequence.

9. A method of producing translation template mRNA used in a cell-free protein synthesis system, the method comprising a step of:
synthesizing translation template mRNA by using the transcription template DNA according to claim 8 in the absence of cells and under the presence of an element used for transcribing transcription template DNA to mRNA.

10. A method of producing a protein, the method comprising a step of:
synthesizing a protein by using the translation template mRNA according to any one of claims 1 to 7 or translation template mRNA produced in the method of producing translation template mRNA according to claim 9 in the absence of cells and under the presence of an element used for translating translation template mRNA into a protein.
